# EUROPEAN PATENT APPLICATION

(11) **EP 4 821 464 A2**
(43) Date of publication of application: **14.10.2026**
(21) Application number: 26193203.2
(22) Date of filing: 08.10.2010
(51) Int. Cl.: A61F 2/856

(54) **STENT GRAFT**

(30) Priority: 09.10.2009 US 25031309 P; 18.06.2010 US 81857510; 18.06.2010 US 81855110
(62) Document number(s) of the earlier application(s) in accordance with Art. 76 EPC: 24189884.0 / 4 464 277; 19192395.2 / 3 610 832; 16164771.4 / 3 067 014; 10768655.2 / 2 485 681
(71) Applicant: W. L. Gore & Associates, Inc., Newark, DE 19711 (US)
(72) Inventor: DAUGHERTY, John R., Arizona, 86004 (US); HAGAMAN, Logan R., Arizona, 86004 (US); HARTMAN, Cody L., Newark, 19711 (US); JACOBY, Russell L., Arizona, 86004 (US); KOVACH, Larry J., Arizona, 86004 (US); WOLFE, Roark N., Arizona, 86004 (US)
(74) Representative: HGF

(57) **Abstract**

A stent graft comprising a graft and a stent, the stent having undulations defining sets of apices extending in first and second opposing directions. The stent graft has a tape member wound helically around the graft to cover the first set of apices and maintain the second set of apices uncovered. The stent graft is configured to be longitudinally compressible to form pleats.

## Description

### CROSS-REFERENCE TO RELATED APPLICATIONS

This application claims benefit to US Provisional Patent Application Serial No. 61/250,313 filed 9 October 2009; US Patent Application Serial No. 12/818,551 filed 18 June 2010; and US Patent Application Serial No. 12/818,575 filed 18 June 2010 which are incorporated by reference herein for all purposes.

### FIELD OF THE INVENTION

One aspect of the invention is directed to an improved, modular, bifurcated stent graft having an integral support tube. Another aspect of the invention is directed to a highly conformable stent graft with an optional bifurcation.

### BACKGROUND

Aneurysms occur in blood vessels at sites where, due to age, disease or genetic predisposition of the patient, the strength or resilience of the vessel wall is insufficient to prevent ballooning or stretching of the wall as blood passes through. If the aneurysm is left untreated, the blood vessel wall may expand and rupture, often resulting in death.

To prevent rupturing of an aneurysm, a stent graft may be introduced into a blood vessel percutaneously and deployed to span the aneurysmal sac. Stent grafts include a graft fabric secured to a cylindrical scaffolding or framework of one or more stents. The stent(s) provide rigidity and structure to hold the graft open in a tubular configuration as well as the outward radial force needed to create a seal between the graft and a healthy portion of the vessel wall and provide migration resistance. Blood flowing through the vessel can be channeled through the luminal surface of the stent graft to reduce, if not eliminate, the stress on the vessel wall at the location of the aneurysmal sac. Stent grafts may reduce the risk of rupture of the blood vessel wall at the aneurysmal site and allow blood to flow through the vessel without interruption.

However, various endovascular repair procedures such as the exclusion of an aneurysm require a stent graft to be implanted adjacent to a vascular bifurcation. Often the aneurysm extends into the bifurcation requiring the stent graft to be placed into the bifurcation. A bifurcated stent graft is therefore required in these cases. Modular stent grafts, having a separate main body and branch component are often preferred in these procedures due to the ease and accuracy of deployment. See U.S. Patent Application No. 2008/0114446 to Hartley et al. for an example of a modular stent graft having separate main body and branch stent components. In the Hartley *et al.* publication the main body stent has a fenestration in the side wall that is tailored to engage and secure the side branch stent. The side branch stent in such a configuration is in a "line to line" interference fit with the main body fenestration, causing a potential compromise to the fatigue resistance of the stent to stent junction. U.S. Patent No. 6,645,242 to Quinn presents a more robust stent to stent joining configuration. In the Quinn patent, a tubular support, internal to the main body stent, is incorporated to enhance the reliability of the stent to stent joining. The tubular, internal support of Quinn provides an extended sealing length along with improved fatigue resistance. However, the innermost tube is made by adding additional material shaped into a tube and sewn and/or adhered to the main graft component.

In addition, Aneurysms occurring in the aorta, the largest artery in the human body, may occur in the chest (thoracic aortic aneurysm) or in the abdomen (abdominal aortic aneurysm). Due to the curvature of the aortic arch, thoracic aortic aneurysms can be particularly challenging to treat. Other parts of the vasculature, such as the common iliac artery which extends from the aorta, can also be extremely tortuous. Hence, a stent graft deployed into such regions is preferably able to conform to the vasculature. The high degree of conformability allows the stent graft to bend and optimally oppose and seal against the native vessel.

### SUMMARY OF THE INVENTION

The one embodiment of the invention is directed to an improved, modular, bifurcated stent graft having an integral support tube. In another embodiment, the invention is directed to a highly conformable stent graft with or without at least one portal for a side branch device (*e.g*. a stent graft).

One embodiment of the invention comprises a multi-lumen stent graft comprising: a primary lumen defined by a graft composed of an innermost tube with an opening and an outermost tube with an opening, said graft being supported by a primary stent; and a secondary lumen disposed between the innermost tube and outermost tube of said graft, wherein said secondary lumen is in fluid communication through said openings. In one embodiment, said secondary lumen comprises a secondary stent or stent assembly. In another embodiment, said secondary lumen can accept another smaller stent graft.

Another embodiment of the invention comprises a stent graft for implantation in a bifurcated body lumen having a main branch vessel and a side branch vessel, wherein the stent graft comprises: a graft, said graft composed of an innermost tube with an opening and an outermost tube with an opening, said graft extending along a longitudinal axis from a distal end to a proximal end and defining a main lumen extending therethrough, said graft being supported by a primary stent; and a secondary lumen disposed between the innermost tube and outermost tube of said graft, said secondary lumen portion positioned between the distal and proximal ends of said graft, wherein said secondary lumen is in fluid communication through said openings of said innermost and outermost tubes. In one embodiment, said primary stent is a self expanding stent.

Another embodiment of the invention comprises covering a first mandrel that comprises a groove and a back wall of said groove with an innermost polymeric tube; slitting said polymeric tube along said back wall of said groove; placing a second mandrel into said groove of the first mandrel and aligning said second mandrel with the back wall of the groove, deforming said innermost polymeric tube; placing an outermost polymeric tube over said inner most tube; and making an opening over said second smaller mandrel; wherein said outermost tube and innermost tube comprise a graft member.

Another embodiment of the invention comprises a graft being supported by a stent, wherein said stent comprise undulations each which comprise apices in opposing first and second directions, and a tape member, having first and second longitudinal edges, attached to said stent and to said graft such that the first tape edge substantially covers the apices in the first or the second direction of the each of the undulations, thus confining the apices in the first direction or second direction of the undulations to the graft and wherein the apices in the first or the second direction of the undulation are not confined relative to the graft. In one embodiment, said apices in the first direction are confined to the graft and the apices in second direction are not confined relative to the graft. In another embodiment, said apices in the second direction apices are confined to the graft and the apices in the first direction are not confined relative to the graft. In another embodiment, said graft forms circumferentially oriented unidirectional pleats where longitudinally compressed. In another embodiment, said apices in the first direction of said undulation are positioned under an adjacent pleat where compressed. In another embodiment, said stent is formed from a single continuous wire helical wrapped around said graft. In another embodiment, said stent is a self-expanding stent. In another embodiment, said stent is made from Nitinol. In another embodiment, said undulations have a sinusoidal shape. In another embodiment, said graft comprises polytetrafluoroethylene.

Additional features and advantages of the invention will be set forth in the description or may be learned by practice of the invention. These features and other advantages of the invention will be realized and attained by the structure particularly pointed out in the written description and claims hereof as well as the appended drawings.

It is to be understood that both the foregoing general description and the following detailed description are exemplary and explanatory and are intended to provide further explanation of the invention as claimed.

### BRIEF DESCRIPTION OF THE DRAWINGS

The accompanying drawings are included to provide a further understanding of the invention and are incorporated in and constitute a part of this specification, illustrate embodiments of the invention and together with the description serve to explain the principles of the invention.

In the drawings:
Figure 1A is a perspective view of a modular, bifurcated stent graft having a main body stent, an internal support tube and attached side branch stent. Figures 1B, 1C, 1D and 1E comprise a bifurcated stent graft, in which the main body comprises at least one side branch portal made from a portion of the main body graft.
Figures 2A and 2B depict perspective views of a mandrel used to construct a main body stent graft having an integral support tube.
Figures 3A and 3B depict perspective views of a mandrel used to construct a main body stent graft having an integral support tube and a secondary stent assembly.
Figures 4A and 4B depict schematic side views of a mandrel used to construct a main body stent graft having an integral support tube and a secondary stent assembly.
Figures 5A, 5B, 5C and 5D are side views of a mandrel and stent fabrication process.
Figure 6 is a top view of a bifurcated stent graft with a side branch portal.
Figure 7 is a perspective view of a side branch stent having three purpose built portions.
Figure 8 depicts a fully extended stent graft.
Figure 9 depicts a flexible stent graft in a state of full longitudinal compression, wherein the unidirectional pleats are formed around the full circumference of the stent graft.
Figures 10A and B depict a partial cross-sectional view of one wall of the stent graft, taken along cross-sectional plane 3-3 of Figure 8, illustrating the unidirectional pleating of the compressed stent graft.
Figure 11 depicts a flexible stent graft a state of partial longitudinal compression (or in a bent shape), wherein the unidirectional pleats are formed on a portion of the stent graft circumference (or on the inner meridian) and the outer meridian has un-pleated or straight graft portions.
Figure 12 depicts a "flat or unrolled" drawing of the cylindrical mandrel.
Figure 13 depicts a single circumference winding pattern.
Figure 14 depicts a stent graft having an undulating, helical wire stent surrounding a graft material. The stent is attached to the graft material by a helical a tape member.

### DETAILED DESCRIPTION OF THE ILLUSTRATED EMBODIMENTS

One embodiment of the invention is directed to an improved, modular, bifurcated stent graft having an integral support tube. In another embodiment, the invention is directed to a highly conformable stent graft with or without at least one portal for a side branch device (*e.g*. a stent graft).

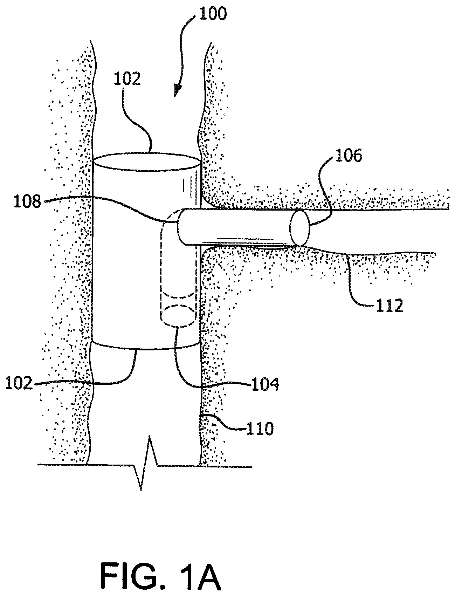

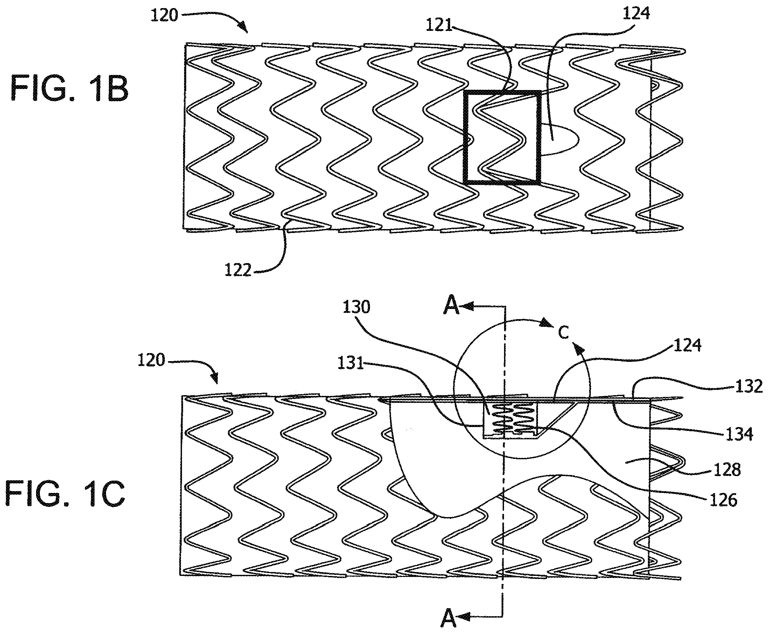

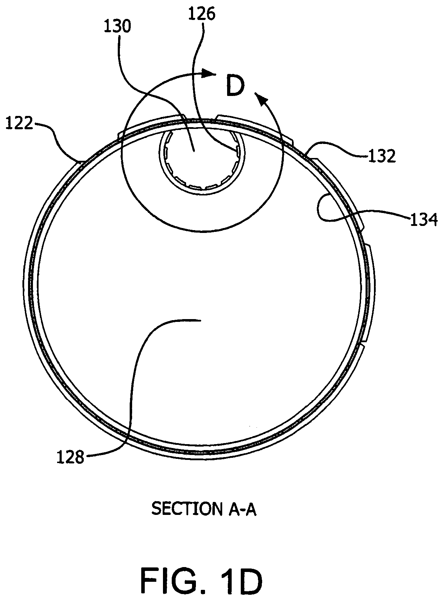

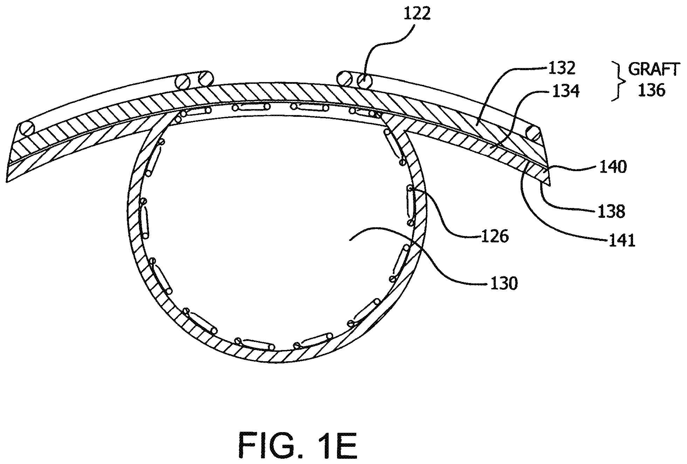

In general, most bifurcated stent grafts have an internal tube to create the bifurcation or a fenestration on the side of a stent graft in which another tube or stent graft is inserted. See, for example U.S. Patent 6,645,242 to Quinn and U.S. Patent 6,077,296 to Shokoohi. **Figure 1** is a perspective view of a general modular bifurcated stent graft **100** having a main body **102** with an internal tube **104**. In general, most internal tubes (i.e. bifurcation tubes) are made by adding additional material that is formed into a tube or a bifurcation site and sewn and/or adhered to the internal side of the main body (usually the graft). The internal tube **104** is sized to engage and secure a side branch device **106**, shown protruding from a main body portal **108**. The main body **102** is shown implanted into a main vessel **110** with the side branch stent implanted into a branch vessel **112**. The instant invention, as depicted in **Figures 1B** to **7**, comprises a bifurcated stent graft, in which the main body comprises at least one side branch portal made from a portion of the main body graft wherein said at least a portion of said portal is integral with said graft and which at least a portion of said portal has no seams in the main blood flow surface of the graft and/or weakened areas due to non-continuous construction.

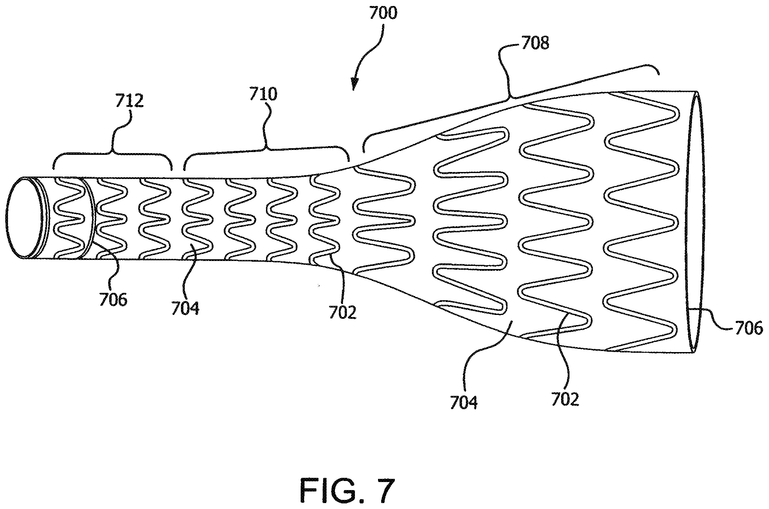

One embodiment of the invention is shown in **Figures 1B** to **1D**. **Figure 1B** is a top view of a bifurcated stent graft **120** having a primary stent (or main body stent) **122** with a side branch portal **124**. Also depicted is a stent feature **121** which creates an area for the side branch portal. In this embodiment, said feature is called the "double W". In this embodiment, said "double W" helps support the side branch portal and prevents said portal from collapsing. In addition, this design creates a region for a side branch portal without creating a high strain region in the body winding pattern of the stent. Without being bound to a particular theory, one reason may be that the "double W" design does not rely on shorter amplitude struts that stiffen the frame and results in higher stains, which may cause fractures when the stent is stressed. The main body portal **124** is sized to engage and secure a side branch stent, one embodiment of which is depicted in **Figure 7**, **700**.

**Figure 1C** is a side view with a partial longitudinal cross section of **Figure 1B**. This Figure depicts primary lumen **128**, a secondary lumen **130**, an outermost tube **132**, an innermost tube **134** and an optional secondary stent **126**. Also depicted is the innermost tube opening **131**.

**Figure 1D** is a cross section of A-A in **Figure 1C**. This Figure depicts primary stent **122**, secondary stent **126**, primary lumen **128**, and secondary lumen **130**. This Figure also depicts an outermost tube **132** and an innermost tube **134**.

**Figure 1E** is a close up of section D depicted on **Figure 1D**. Thus, this Figure is a close up of the cross section of the side branch portal. This Figure depicts the primary stent **122**, secondary stent **126**, and secondary lumen **130**. This Figure also depicts an outermost tube **132** and an innermost tube **134**. Graft **136** is composed of innermost tube **134** and outermost tube **132**. Also depicted is the blood flow surface 138 (i.e. the internal graft surface), the outer surface of the innermost tube **140** and the inner surface of the outermost tube **141**.

Thus, one embodiment of the invention, the bifurcated (multi-lumen) stent graft, comprises a primary lumen **128** defined by a graft **136** composed of an innermost tube **134** with an opening **131** and an outermost tube **132** with an opening **124**, said graft being supported by a primary stent **122**; and a secondary lumen **130** disposed between the innermost tube **132** and outermost tube **134** of said graft **136**; wherein said secondary lumen is in fluid communication through said openings **131** and **124**. In one embodiment, said secondary lumen **130** comprises a secondary stent **126** or stent assembly. As used herein, said secondary stent assembly is a secondary stent that is covered and may comprise additional features such as radiopaque markers. In another embodiment, said secondary lumen is disposed between the ends of the main stent graft or main body. In another embodiment, a portion of the said secondary stent or stent assembly abuts against a portion of the innermost tube **134**. In another embodiment, said secondary stent or stent assembly abuts against a portion of graft **136**. In another embodiment, a portion of said secondary stent or stent assembly lays on the outer surface of the innermost tube **140**. In another embodiment, said secondary lumen is defined partially by the innermost tube and partially by the outermost tube. In another embodiment, said secondary lumen is defined partially by the outer surface of the innermost tube **140** and the inner surface of the outermost tube **141**.

The graft of the stent graft of the invention may be made up of any material which is suitable for use as a graft in the chosen body lumen. Said graft can be composed of the same or different materials. Furthermore, said graft can comprise multiple layers of material that can be the same material or different material. Although the graft can have several layers of material, said graft may have a layer that is formed into a tube (innermost tube) and an outermost layer that is formed into a tube (outermost tube). For the purposes on this invention, the outermost tube does not comprise a tape layer that may be used to adhere a stent to a graft as described in more detail below. In one embodiment of the invention, said graft comprises an innermost tube and an outermost tube.

Many graft materials are known, particularly known are those that can be used as vascular graft materials. In one embodiment, said materials can be used in combination and assembled together to comprise a graft. The graft materials used in a stent graft can be extruded, coated or formed from wrapped films, or a combination thereof. Polymers, biodegradable and natural materials can be used for specific applications.

Examples of synthetic polymers include, but are not limited to, nylon, polyacrylamide, polycarbonate, polyformaldehyde, polymethylmethacrylate, polytetrafluoroethylene, polytrifluorochlorethylene, polyvinylchloride, polyurethane, elastomeric organosilicon polymers, polyethylene, polypropylene, polyurethane, polyglycolic acid, polyesters, polyamides, their mixtures, blends and copolymers are suitable as a graft material. In one embodiment, said graft is made from a class of polyesters such as polyethylene terephthalate including DACRON^{®} and MYLAR^{®} and polyaramids such as KEVLAR^{®}, polyfluorocarbons such as polytetrafluoroethylene (PTFE) with and without copolymerized hexafluoropropylene (TEFLON^{®} or GORE-TEX^{®}), and porous or nonporous polyurethanes. In another embodiment, said graft comprises expanded fluorocarbon polymers (especially PTFE) materials described in British. Pat. Nos. 1,355,373; 1,506,432; or 1,506,432 or in U.S. Pat. Nos. 3,953,566; 4,187,390; or 5,276,276, the entirety of which are incorporated by reference. Included in the class of preferred fluoropolymers are polytetrafluoroethylene (PTFE), fluorinated ethylene propylene (FEP), copolymers of tetrafluoroethylene (TFE) and perfluoro (propyl vinyl ether) (PFA), homopolymers of polychlorotrifluoroethylene (PCTFE), and its copolymers with TFE, ethylene-chlorotrifluoroethylene (ECTFE), copolymers of ethylene-tetrafluoroethylene (ETFE), polyvinylidene fluoride (PVDF), and polyvinyfluoride (PVF). Especially preferred, because of its widespread use in vascular prostheses, is ePTFE. In another embodiment, said graft comprises a combination of said materials listed above. In another embodiment, said graft is substantially impermeable to bodily fluids. Said substantially impermeable graft can be made from materials that are substantially impermeable to bodily fluids or can be constructed from permeable materials treated or manufactured to be substantially impermeable to bodily fluids (*e.g.* by layering different types of materials described above or known in the art). In another embodiment, said outermost tube comprises ePTFE. In another embodiment, said innermost tube comprises ePTFE. In another embodiment, said innermost and outermost tube comprises ePTFE film that has been wrapped into a tube. In another embodiment, said secondary stent is covered with any of the material disclosed herein or known in the art. In another embodiment, the secondary stent covering comprises ePTFE.

Additional examples of graft materials include, but are not limited to, vinylidinefluoride/hexafluoropropylene hexafluoropropylene (HFP), tetrafluoroethylene (TFE), vinylidenefluoride, 1-hydropentafluoropropylene, perfluoro (methyl vinyl ether), chlorotrifluoroethylene (CTFE), pentafluoropropene, trifluoroethylene, hexafluoroacetone, hexafluoroisobutylene, fluorinated poly(ethylene-co-propylene (FPEP), poly(hexafluoropropene) (PHFP), poly(chlorotrifluoroethylene) (PCTFE), poly(vinylidene fluoride (PVDF), poly(vinylidene fluoride-co-tetrafluoroethylene) (PVDF-TFE), poly(vinylidene fluoride-co-hexafluoropropene) (PVDF-HFP), poly(tetrafluoroethylene-co-hexafluoropropene) (PTFE-HFP), poly(tetrafluoroethylene-co-vinyl alcohol) (PTFE-VAL), poly(tetrafluoroethylene-co-vinyl acetate) (PTFE-VAC), poly(tetrafluoroethylene-co-propene) (PTFEP) poly(hexafluoropropene-co-vinyl alcohol) (PHFP-VAL), poly(ethylene-co-tetrafluoroethylene) (PETFE), poly(ethylene-co-hexafluoropropene) (PEHFP), poly(vinylidene fluoride-co-chlorotrifluoroe- thylene) (PVDF-CTFE), and combinations thereof, and additional polymers and copolymers described in U.S. Publication 2004/0063805, incorporated by reference herein in its entirety for all purposes. Additional polyfluorocopolymers include tetrafluoroethylene (TFE)/perfluoroalkylvinylether (PAVE). PAVE can be perfluoromethylvinylether (PMVE), perfluoroethylvinylether (PEVE), or perfluoropropylvinylether (PPVE), as essentially described in U.S. Publication 2006/0198866 and U.S. Patent 7,049,380, both of which are incorporated by reference herein for all purposes in their entireties. Other polymers and copolymers include, polylactide, polycaprolacton-glycolide, polyorthoesters, polyanhydrides; poly-aminoacids; polysaccharides; polyphosphazenes; poly(ether-ester) copolymers, e.g., PEO-PLLA, or blends thereof, polydimethyl-siolxane; poly(ethylene-vingylacetate); acrylate based polymers or copolymers, e.g., poly(hydroxyethyl methylmethacrylate, polyvinyl pyrrolidinone; fluorinated polymers such as polytetrafluoroethylene; cellulose esters and any polymer and copolymners described in U.S. Publication 2004/0063805, incorporated by reference herein in its entity.

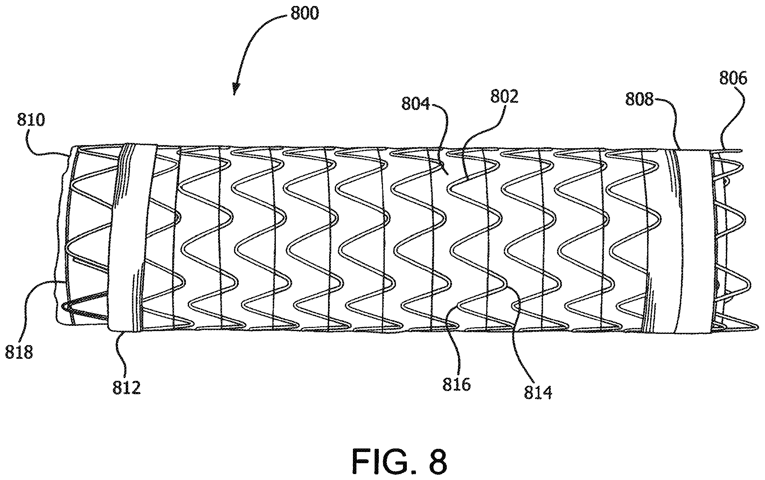

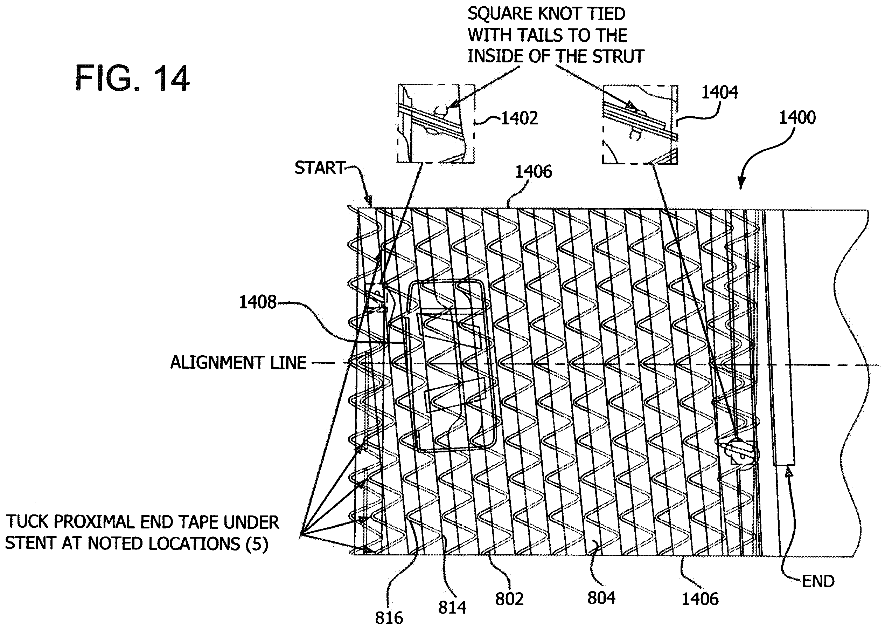

Said stents of the instant intention are generally cylindrical and comprise helically arranged undulations having plurality of helical turns. The undulations preferably are aligned so that they are "in-phase" with each other as shown in **Figure 8**. More specifically, undulations comprise apices in opposing first **814** and second **816** directions. When the undulations are in-phase, apices in adjacent helical turns are aligned so that apices can be displaced into respective apices of a corresponding undulation in an adjacent helical turn. In one embodiment, said undulations have a sinusoidal shape. In another embodiment, said undulations are U shaped. In another embodiment, said undulations are V shaped. In another embodiment, said undulations are ovaloid shaped. These shapes are fully described in U.S. Patent 6,042,605, **Figures 14A-E**. U.S. Patent 6,042,605 is incorporated by reference herein in its entirety for all purposes.

In another embodiment of the invention, said stent can be fabricated from a variety of biocompatible materials including commonly known materials (or combinations of materials) used in the manufacture of implantable medical devices. Typical materials include 316L stainless steel, cobalt-chromium-nickel-molybdenum-iron alloy ("cobalt-chromium"), other cobalt alloys such as L605, tantalum, Nitinol, or other biocompatible metals. In one embodiment, said stent graft is a balloon expandable stent graft. In another embodiment, said stent graft is a self-expanding stent graft. In another embodiment, said stent is a wire wound stent. In another embodiment, said wire wound stent comprise undulations.

The wire wound stent can be constructed from a reasonably high strength material, *i.e*., one which is resistant to plastic deformation when stressed. In one embodiment, the stent member comprises a wire which is helically wound around a mandrel having pins arranged thereon so that the helical turns and undulations can be formed simultaneously, as described below. Other constructions also may be used. For example, an appropriate shape may be formed from a flat stock and wound into a cylinder or a length of tubing formed into an appropriate shape or laser cutting a sheet of material. In another embodiment, said stent is made from a super-elastic alloy. There are a variety of disclosures in which super-elastic alloys such as nitinol are used in stents. See for example, U.S. Pat. Nos. 4,503,569, to Dotter; 4,512,338, to Balko et al.; 4,990,155, to Wilkoff; 5,037,427, to Harada, et al.; 5,147,370, to MacNamara et al.; 5,211,658, to Clouse; and 5,221,261, to Termin et al.

A variety of materials variously metallic, super elastic alloys, such as Nitinol, are suitable for use in these stents. Primary requirements of the materials are that they be suitably springy even when fashioned into very thin sheets or small diameter wires. Various stainless steels which have been physically, chemically, and otherwise treated to produce high springiness are suitable as are other metal alloys such as cobalt chrome alloys (*e.g*., ELGILOY^{®}), platinum/tungsten alloys, and especially the nickel-titanium alloys generically known as "nitinol".

Nitinol is especially preferred because of its "super-elastic" or "pseudoelastic" shape recovery properties, *i.e*., the ability to withstand a significant amount of bending and flexing and yet return to its original form without permanent deformation. These metals are characterized by their ability to be transformed from an austenitic crystal structure to a stress-induced martensitic structure at certain temperatures, and to return elastically to the austenitic shape when the stress is released. These alternating crystalline structures provide the alloy with its super-elastic properties. These alloys are well known but are described in U.S. Pat. Nos. 3,174,851; 3,351,463; and 3,753,700.

Other suitable stent materials include certain polymeric materials, particularly engineering plastics such as thermotropic liquid crystal polymers ("LCP's"). These polymers are high molecular weight materials which can exist in a so-called "liquid crystalline state" where the material has some of the properties of a liquid (in that it can flow) but retains the long range molecular order of a crystal. The term "thermotropic" refers to the class of LCP's which are formed by temperature adjustment. LCP's may be prepared from monomers such as p,p'-dihydroxy-polynuclear-aromatics or dicarboxy-polynuclear-aromatics. The LCP's are easily formed and retain the necessary interpolymer attraction at room temperature to act as high strength plastic artifacts as are needed as a foldable stent. They are particularly suitable when augmented or filled with fibers such as those of the metals or alloys discussed below. It is to be noted that the fibers need not be linear but may have some preforming such as corrugations which add to the physical torsion enhancing abilities of the composite.

Another embodiment of the invention comprises a stent graft for implantation in a bifurcated body lumen having a main branch vessel and a side branch vessel, wherein the stent graft comprises: a graft, said graft composed of an innermost tube with an opening and an outermost tube with an opening, said graft extending along a longitudinal axis from a distal end to a proximal end and defining a main lumen extending therethrough, said graft being supported by a primary stent; and a secondary lumen disposed between the innermost tube and outermost tube of said graft, said secondary lumen portion positioned between the distal and proximal ends of said graft, said secondary lumen is in fluid communication through said openings of said innermost and outermost tubes. In one embodiment, said primary stent is a self expanding stent. In another embodiment, said self expanding stent comprises a titanium-nickel alloy. In another embodiment, said stent comprises a single continuous wire helically wrapped around said graft. In another embodiment, wherein said single continuous wire comprises undulations. In another embodiment, said undulating wire comprises multiple turns of said undulations, and each turn of said undulating wire comprises multiple apexes, with undulation in one turn generally in-phase with undulation in an adjacent turn. In another embodiment, said undulations are U shaped. In another embodiment, said undulations are V shaped. In another embodiment, said undulations are ovaloid shaped. In another embodiment, said undulations are sinusoidal shaped. In another embodiment, said stent is attached to said graft. In another embodiment, said stent is attached to said graft by a ribbon or tape. In another embodiment, said ribbon or tape is adhered to a portion of said stent and a portion of said graft. In another embodiment, said ribbon or tape is arranged in a helical configuration with multiple turns. In another embodiment, said ribbon or tape is arranged in a helical configuration with multiple turns, each turn being spaced from an adjacent turn. In another embodiment, said spacing between said turns is uniform. In another embodiment, said ribbon covers a portion of said undulation. In another embodiment, said stent comprises undulations each which comprise an apex portion and a base portion and said ribbon or tape is attached to said stent such that the ribbon is placed along to the base portion of the each of the undulations thus confining the base portion of the undulations to the graft and wherein the apex portion of the undulation is not confined.

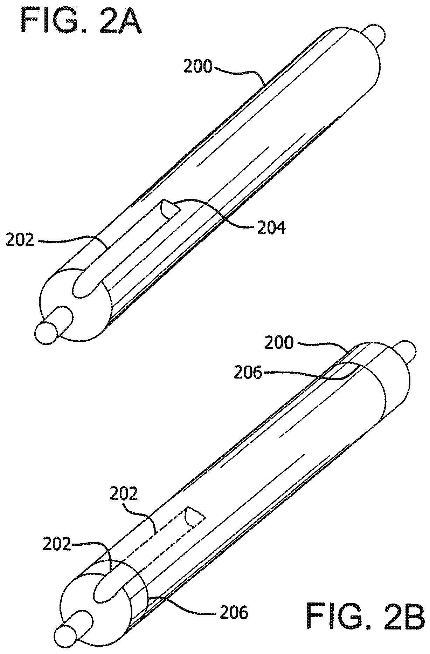

At least one method of making a main body stent graft having an integral support tube is described in **Figure 2** through **Figure 7**.

**Figure 2A** is a perspective view of a metallic mandrel **200** having a slot or groove **202** formed into one end of the mandrel. The groove **202** terminates into a back wall **204**. As shown in perspective view **Figure 2B**, an inner tube **206** is slip-fit over the mandrel **200**, covering a portion of the mandrel groove **202**. An inner tube can comprise any biocompatible polymer that is deformable (to allow a subsequent insertion of a side branch stent) and can be extruded, coated or formed from wrapped films. Suitable materials used for the inner tube may include, but are not limited to, any of the material described above, any other biocompatible material commonly known in the art or a combination thereof.

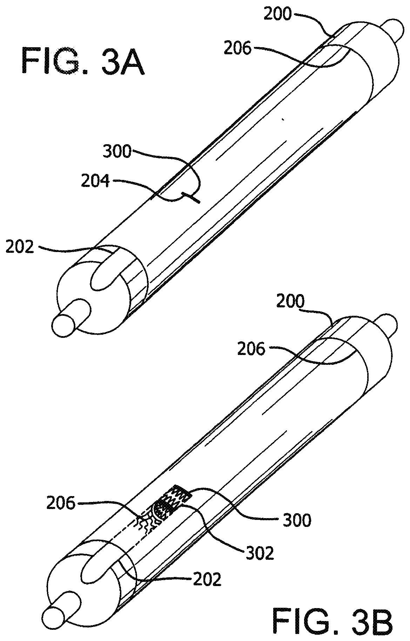

**Figure 3A** is a perspective view of the mandrel **200** covered by the inner tube **206**. The inner tube is cut, forming a slit **300** at the back wall **204** of the mandrel groove **202**. As shown in **Figure 3B**, a side branch or secondary stent assembly **302** is aligned to the mandrel groove **202**, mandrel back wall **204** and inner tube slit **300**. A first support segment (subsequently described) is placed into the secondary stent assembly and the secondary stent assembly **302** (with the first support segment) is then inserted into the mandrel groove **202**, deforming the inner tube **206** into the mandrel groove. The back wall **204** defines the opening in the innermost tube (**131**, **Figure 1E**)

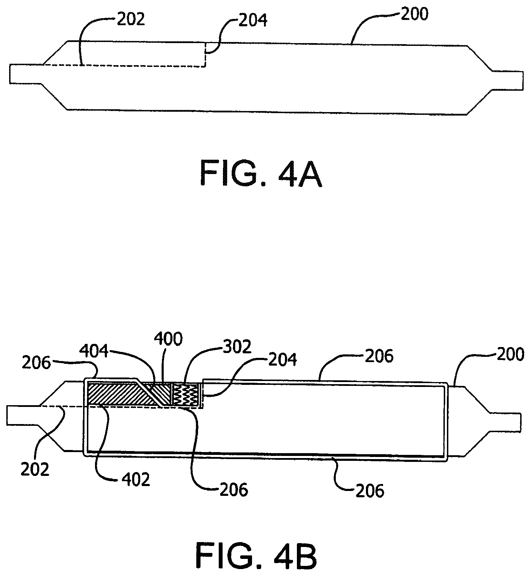

To control the deformed shape of the inner tube, support segments are placed into the secondary stent assembly and into the mandrel groove, as depicted in **Figures 4A** and **4B**. Shown in **Figure 4A** is a side view schematic of the mandrel **200**, the groove **202** and the groove back wall **204**. Shown in **Figure 4B** is a side view schematic of the mandrel **200**, mandrel groove **202** and inner tube **206**. The secondary stent assembly **302** has been placed over a first support segment **400** having an end formed to mate to the mandrel groove back wall **204**. The opposing end of the first support segment has a tapered or angulated wall as depicted in **Figure 4B**. A second support segment **402** is placed into the mandrel groove **202** under the inner tube **206**. The second support structure can have an angulated wall that mates with the angulated wall of the first support structure, although it is not required for the second support structure to have an angulated wall. One of the purposes of this second support structure to keep first support segment **400** in place during manufacturing. The inner tube **206** is shown deformed into the mandrel groove **202**. The inner tube **206** is also shown having a tapered, beveled or angulated wall portion **404** formed by the angulated wall of the support segment **400**.

To further strengthen the inner tube (**Figure 3A**, **206**), additional sheet or film layers may be added onto the inner tube prior to the insertion of the secondary stent assembly. For example a square/rectangle shaped thin film sheet having a high degree of bi-axial strength may be placed onto the inner tube **206** and aligned to the mandrel groove. The sheet can be dimensioned to be wider than the mandrel groove width and have a length approximating the mandrel groove length. This strengthening layer will then be deformed into the mandrel groove, providing additional support to the inner tube/secondary stent assembly. Multiple strengthening layers may be combined to enhance the properties of the inner tube. Suitable materials used for strengthening layers may include, but are not limited to, any of the material described above, any other biocompatible material commonly known in the art or a combination thereof.

Although the above methods describe the making of a bifurcated stent graft with only one portal, additional portals can also be made using similar methods describe above. Thus, another embodiment of the invention comprises a stent graft with at least two portals. In another embodiment, said stent graft of the invention comprises three, four, five, six or seven portals. Such a stent graft may be useful for, *inter alia*, implanting a stent graft in the abdominal aorta where the renal arteries branch off. In addition, due to the stent graft of the invention being highly conformable, see below, said stent graft of the invention with three portals can be placed in the arch of the aorta without blocking blood flow to the left subclavian artery, left common carotid artery and the bachiocephalic artery. In another embodiment, said several portals can be placed where desired longitudinally along the stent and/or circumferentially around the stent. A person of skill in the art can design said portals at any region in the vasculature.

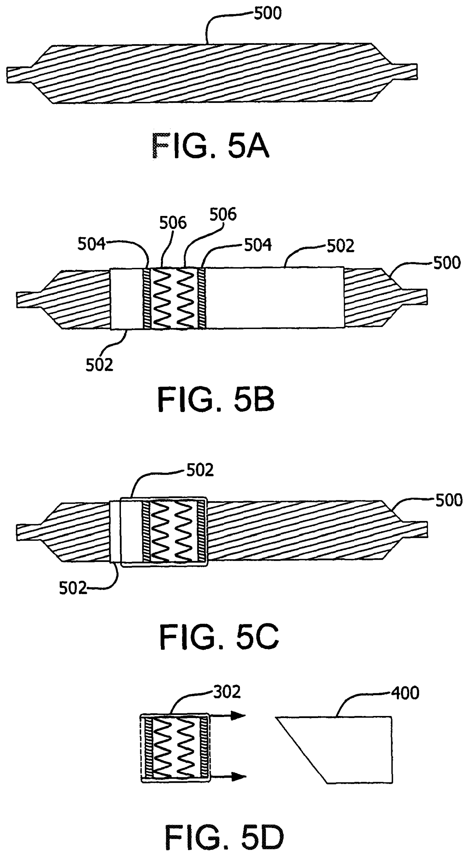

At least one method of making a secondary stent assembly is outlined in **Figures 5A** through **5D**. As shown in side view **Figures 5A** and **5B**, a polymeric tube **502** is slip-fit onto a mandrel **500**. An undulating wire can be formed into a ring stent **506** by winding the wire onto a mandrel with protruding pins. The diameters of the mandrel and pins along with the locations of the pins dictate the final configuration of the ring stent. After the wire is wound onto the mandrel, the mandrel and wire are heat treated and quenched to set the shape of the stent. The wire is then removed from the mandrel. The ends of the wire are joined together with a section of polymeric heat shrink tubing, forming ring stent **506**. Other methods can be used to make the secondary stent (*e.g*. laser cutting). One or more of these ring stents **506** are then placed onto the polymeric tube **502**. Optional radiopaque marker bands **504** are then placed onto the polymeric tube **502**. The wire or metal tube used to make the secondary stent is described above. In one embodiment, said secondary stent comprises Nitinol.

Next, as shown in **Figure 5C**, one end of the polymeric tube **502** is inverted and drawn over the wire ring stents **506** and optional radiopaque marker bands **504**. The mandrel, polymeric tube, ring stents and radiopaque bands are then heat treated to bond the components together into a stent assembly. The assembly is removed from the mandrel and trimmed to length, forming a secondary stent assembly **302** as shown in **Figure 5D**. The secondary stent assembly is then placed onto a first support segment **400**. Radiopaque markers include, but are not limited to gold, platinum, platinum-tungsten, palladium, platinum-iridium, rhodium, tantalum, or alloys or composites of these metals.

As previously described in **Figure 4B**, the secondary stent (or secondary stent assembly) and the first support assembly are then inserted into the mandrel groove **202**, deforming the inner tube **206** into the mandrel groove. The assembly shown in **Figure 4B** is then covered with an outer polymeric tube. As described herein, said tube can be can be extruded, coated or formed from wrapped films.

The assembly is heat treated to join the inner tube to the outer tube. A side branch portal or opening (**Figure 1**, item **108**) is formed as described above. A primary wire stent is then formed by winding a wire onto a mandrel with protruding pins. The wire is heat treated to set the shape of the wire with a process similar to that used to form the secondary stent (**Figure 5B**). The primary stent is then placed over the outer polymeric tube and overwrapped with a polymeric film. The assembly is then heat treated to bond the components together.

Methods of attaching a stent to a graft are known in the art. One embodiment comprises a coupling member that is generally a flat ribbon or tape having at least one generally flat surface. In another embodiment of the invention, the tape member is made from expanded PTFE (ePTFE) coated with an adhesive. In another embodiment, said adhesive is a thermoplastic adhesive. In another embodiment, said thermoplastic adhesive is fluorinated ethylene propylene (FEP). In this embodiment, the FEP-coated side faces toward and contacts the exterior surface of the stent and graft, thus attaching the stent to the graft. Although a particular tape member configuration and pattern has been illustrated and described, other configuration and/or patterns may be used without departing from the scope of the present invention. Materials and method of attaching a stent to the graft is discussed in U.S. Patent 6,042,602 to Martin, incorporated by reference herein for all purposes.

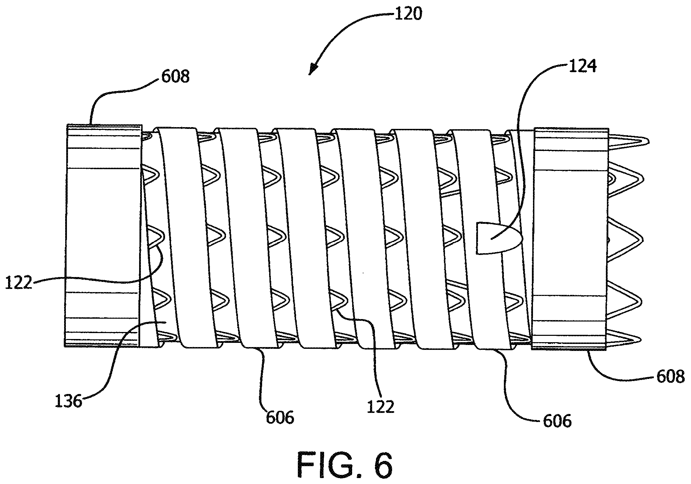

**Figure 6** depicts a top view of a bifurcated stent graft **120** with a side branch portal **124**. In this embodiment, the stent graft comprises a helically formed undulating wire primary stent **122**. The primary stent **122** is joined to graft **136** by a film wrapping **606**, as described above. The stent has film wrapped sealing cuffs **608** on the two opposing ends of the stent graft assembly **120**. Such methods of assembly are generally disclosed in, for example, United States Patent No. 6,042,605 issued to Martin, et al., United States Patent No. 6,361,637 issued to Martin, et al. and United States Patent No. 6,520,986 issued to Martin, et al. incorporated by reference herein for all purposes.

A side branch stent graft would ideally have a distal portion having a high degree of radial stiffness to allow apposition and sealing against a vessel wall. The side branch stent would also have a mid-portion that is highly flexible and highly fatigue resistant to the pulsatile and cyclic loading imparted by the native vessels. The side branch stent would also have a proximal portion that is deployed into the main body stent. This proximal portion of the side branch stent requires a high degree of radial stiffness in order to dock and seal properly into the main body portal.

Shown in **Figure 7** is one embodiment of a side branch stent graft **700**, comprising a wire wound metallic stent **702**, a graft covering **704** and radiopaque marker bands **706**. The side branch stent has a distal portion **708**, a mid-portion **710** and a proximal portion **712**. The distal portion **708** has a high degree of radial stiffness to allow apposition and sealing against a branch vessel wall (**Figure 1**, **112**). The mid-portion **710** is highly flexible and highly fatigue resistant to the pulsatile and cyclic loading imparted by the native vessels. The proximal portion **712** that is deployed into the main body stent (**Figure 1**, **102**), has a high degree of radial stiffness in order to dock and seal properly into the main body portal and can resist compression and remain patent if an additional device deployment is used (*e.g*. an extender).

The process used to manufacture a side branch stent graft **700**, can be used to fabricate the stent graft assembly (**Figure 6**, **120**) as defined above. Such methods of assembly are generally disclosed in, for example, United States Patent No. 6,042,605 issued to Martin, et al., United States Patent No. 6,361,637 issued to Martin, et al. and United States Patent No. 6,520,986 issued to Martin, et al. The stiffness, radial strength, flexibility and fatigue life of a side branch stent can be controlled by the stent wire properties, wound pattern geometries of the wire, graft properties and wire to graft attachment configurations. For example in **Figure 7**, the distal portion **708** of the side branch stent **700** has an undulating wire pattern with relatively large undulation amplitude. The undulations are also spaced relatively far apart. In comparison, the mid-portion **710** of the side branch stent has an undulating wire pattern with relatively small undulation amplitude. The undulations are also spaced relatively far apart. Finally, the proximal portion **712** that is deployed into the main body stent (**Figure 1**, **102**), has an undulating wire pattern with relatively large undulation amplitude. The undulations are also spaced relatively close to the adjacent wires.

Methods of joining the side branch stent graft to the main-stent graft are known. These include, but are not limited to friction fits, hooks, and barbs and/or raised stent apices. Additional methods are disclosed in U.S. Publication 2009/0043376 to Hamer and Zukowski, incorporated by reference herein in its entirety for all purposes.

The stent graft may be delivered percutaneously, typically through the vasculature, after having been folded to a reduced diameter. Once reaching the intended delivery site it is expanded to form a lining on the vessel wall. In one embodiment the stent graft is folded along its longitudinal axis and restrained from springing open. The stent graft is then deployed by removing the restraining mechanism, thus allowing the graft to open against the vessel wall. The stent grafts of this invention are generally self-opening once deployed. If desired, an inflatable balloon catheter or similar means to ensure full opening of the stent graft may be used under certain circumstances. In another embodiment, said stent graft is a balloon expandable stent. The side branch can also be delivered percutaneously after having been folded to a reduced diameter.

The stent graft of the invention may comprise at least one or two radiopaque markers, to facilitate proper positioning of the stent graft within the vasculature. Said radiopaque markers can be used to properly align the stent graft both axially and rotationally to confirm that the side portal is properly aligned. Said radio markers include, but are not limited to gold, platinum, platinum-tungsten, palladium, platinum-iridium, rhodium, tantalum, or alloys. Alternatively, provided that the delivery catheter design exhibits sufficient torque transmission, the rotational orientation of the graft maybe coordinated with an indexed marker on the proximal end of the catheter, so that the catheter may be rotated to appropriately align the side branch(es). Additional methods of delivering the bifurcated stent graft of the invention and an associated side branch are disclosed in U.S. Publication 2008/0269866 to Hamer and Johnson and U.S. Publication 2008/0269867 to Johnson, both of which are incorporated by reference herein in their entirety for all purposes.

Another embodiment of the invention comprises a highly conformable stent graft that can conform to highly tortuous sections of a native vessel. Said stent graft may optionally encompass at least one side branch portal.

Referring to **Figure 8**, the highly conformable stent graft of the invention **800** generally includes a graft **804**, a stent **802** and a tape member (**1406**, **Figure 14**) for coupling the stent and graft member together and is highly conformable. Preferably, the stent and graft are coupled together so that they are generally coaxial.

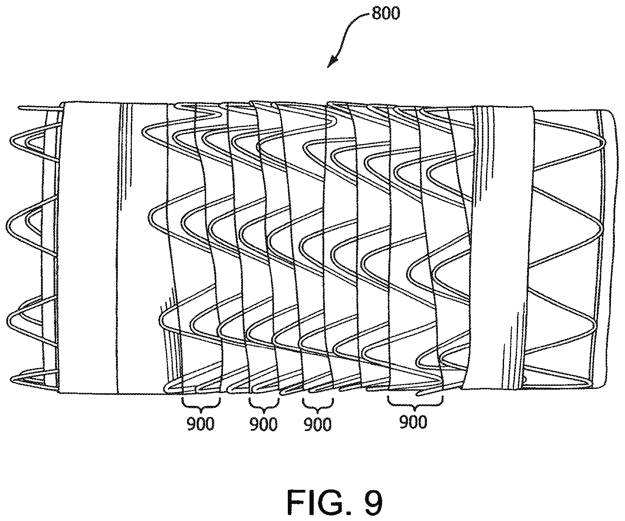

In one embodiment of the invention, the highly conformable stent graft **800** has a helically formed wire stent **802** surrounding a graft **804**. The wire form stent has opposing first **814** and second **816** direction apices. The stent graft **800** has a first end portion **806** optionally comprising a sealing cuff **808**. Similarly, the stent graft **800** has a second end portion **810** optionally comprising a second sealing cuff **812** (folded back for illustration purposes) and a radiopaque marker **818**. As depicted in **Figure 9**, the flexible stent graft **800** has unidirectional pleats **900** that are formed upon longitudinal compression. In one embodiment, said stent graft of the invention has at least one portal between the ends of said stent graft of the invention for the introduction of a side branch device. In another embodiment, said side branch device is a stent graft.

**Figure 9** shows a flexible stent graft **800** in a state of longitudinal compression, wherein the unidirectional pleats **900** are formed around the full circumference of the stent graft **800**.

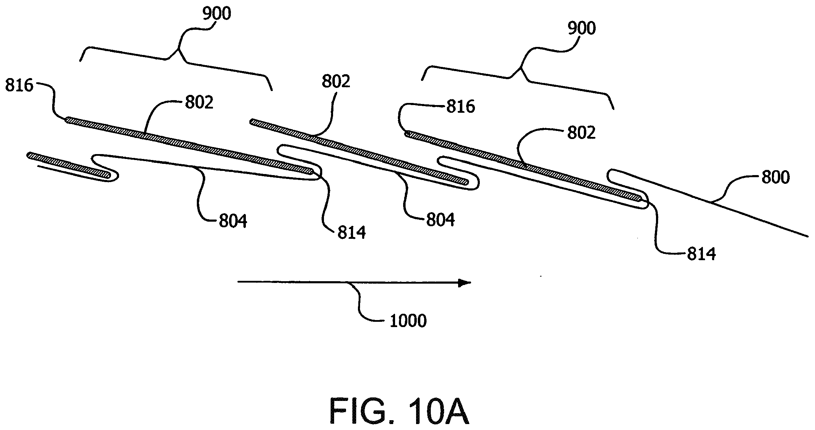

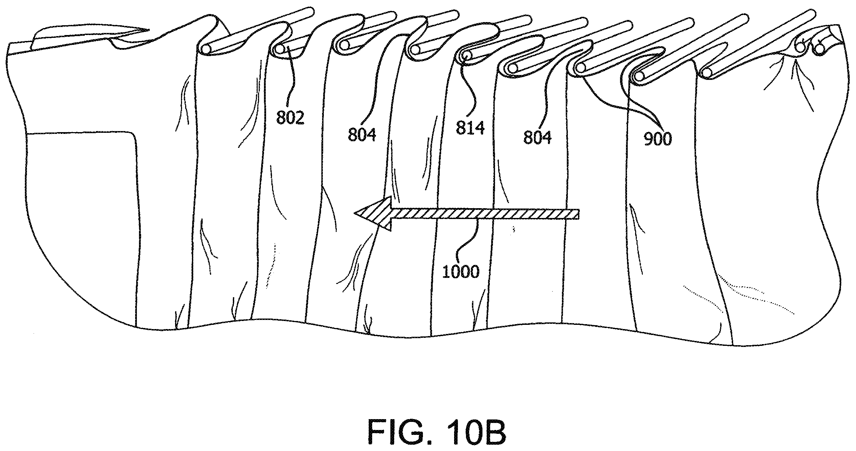

**Figure 10A** is a partial longitudinal cross-sectional view of one wall of the stent graft **800**, taken along cross-sectional plane 3-3 of **Figure 9**, illustrating the unidirectional pleating of the compressed stent graft **800**. The unidirectional pleats have a common orientation and are all bent in the same direction. The wire stent **802** is shown with opposing first directional apices **814** tucked under an adjacent folded portion of the graft material **804**, forming a unidirectional pleat **900**. The arrow **1000** indicates a preferred blood flow direction as "going with the pleats" to minimize flow disruption and turbulence. **Figure 10B** is a long cross-sectional view similar to that of **Figure 10A**, showing unidirectional pleats **900**, along with a preferred blood flow direction **1000**.

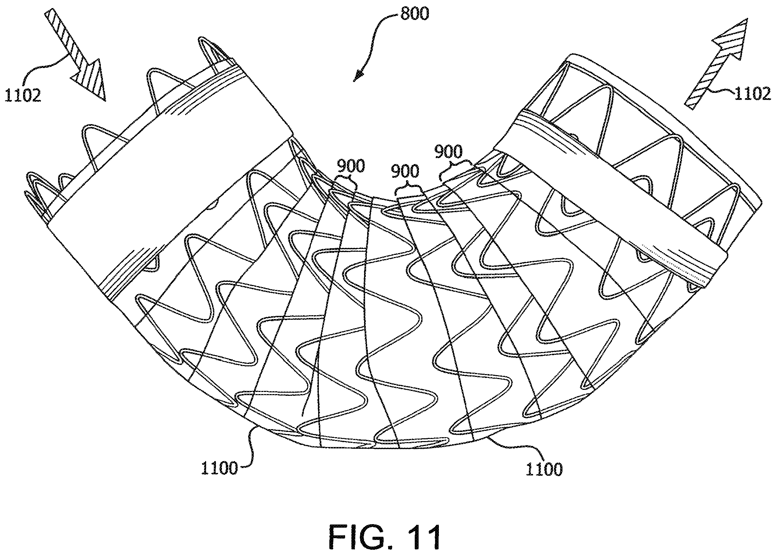

**Figure 11** shows a flexible stent graft **800** in a bent shape that imparts compression to the wall of the graft along the inner meridian of the bend (*i.e*. partial longitudinal compression) wherein the unidirectional pleats **900** are formed on a portion of the stent graft circumference (or the inner meridian). The outer meridian has un-pleated or straight graft portions **1100**. The arrow **1102** indicates a preferred blood flow direction as previously shown in **Figure 10**.

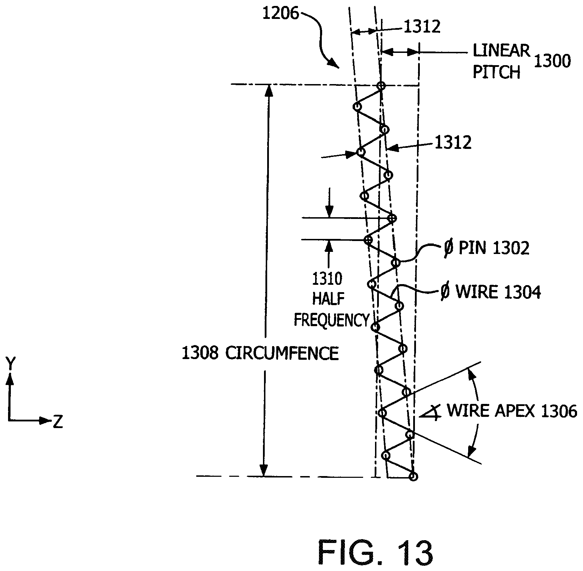

One embodiment of the invention comprises a graft being supported by a stent, wherein said stent comprises undulations each which comprise apices in opposing first and second directions, and a tape member, having first and second longitudinal edges, attached to said stent and to said graft such that the first tape edge substantially covers the apices in the first or the second direction of the each of the undulations, thus confining the apices in the first or the second direction of the undulations to the graft and wherein the apices in the first or the second direction of the undulation are not confined relative to the graft. In one embodiment, said apices in the first direction apices are confined to the graft and the second direction apices are not confined relative to the graft. In another embodiment, said apices in the second direction apices are confined to the graft and the first direction apices are not confined relative to the graft. In another embodiment, said graft forms circumferentially oriented unidirectional pleats where longitudinally compressed. In another embodiment, said confined apices (either in the first direction or second direction) of said undulation are positioned under an adjacent pleat when compressed. The term "confined apices" means that the apices are attached to the graft by either a tape member or attached by another method known in the art. In another embodiment, said confined apices are positioned under an adjacent pleat thereby covering about 1%, about 2%, about 3%, about 4%, about 5%, about 10%, about 20%, about 30%, about 40%, about 50% about 60%, about 70%, about 80% of undulation height **1312** (**Figure 13**) of the apices in the first direction. Depending on the method of taping the stent to the graft, stent design, graft construction and/or any other consideration due to the construction of the stent graft, not all apices may be positioned under an adjacent pleat or may differ in the undulation height **1312** that can be positioned behind an adjacent pleat. Thus, there may be sections of the stent graft that may not be compressible in accordance with the instant invention. Thus, in another embodiment, only a section of the stent graft may be compressed by positioning confined apices under an adjacent pleat. In another embodiment, only a portion of the stent graft may be folded by positioning confined apices under an adjacent pleat (in the inner meridian), as depicted in **Figure 11**. Although the disclosed embodiment comprises the apices in the first direction positioned behind pleats, the invention also encompasses apices in the second direction that are attached to the graft and are positioned under an adjacent pleat, while the apices in the first direction are not confined.

An important aspect of the invention is that the tape member, which comprises a first and second longitudinal edge, secures the stent member to the graft member and covers only a portion of the stent member. Specifically, said tape member is attached to said stent and to said graft such that the first edge of said tape member substantially covers of the apices in the first direction of the each of the undulations, thus confining the apices in the first direction of the undulations to the graft. In one embodiment, the first edge of said tape member is aligned to the edge of the apices in the first direction **814** of the each of the undulations, as essentially depicted in **Figure 14**. With this construction when the stent graft is compressed, the graft forms circumferentially unidirectional pleats and allows said apices in the first direction **814** to be positioned under an adjacent pleat, as shown in **Figures 9** and **11**. The formation of said unidirectional pleats makes said stent graft more conformable, thus giving the stent graft the ability to bend, as depicted in **Figure 11**. In one embodiment, said stent graft can bend to at least 90° without kinking (*i.e*. maintains an essentially circular cross-section in the luminal surface). In another embodiment, said stent graft can bend to at least 90° without kinking after *in-vivo* deployment.

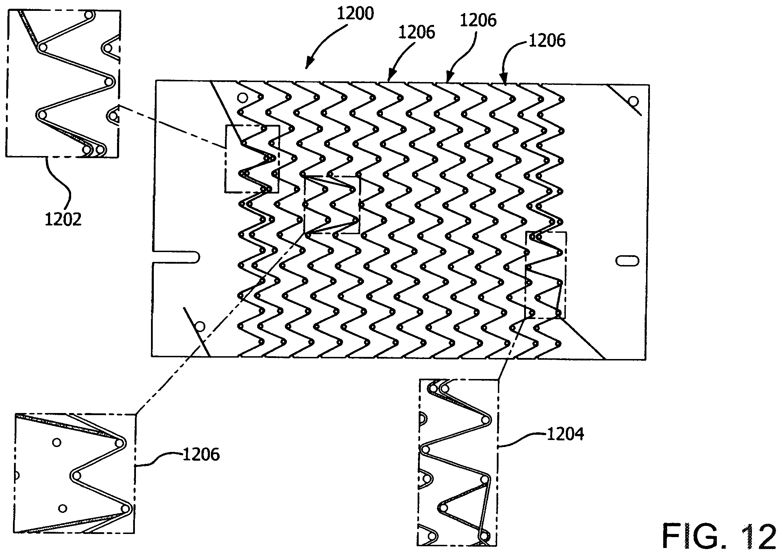

The tape member has a generally broad and/or flat surface for interfacing with the stent and graft. This increases potential bonding surface area between the tape member and the graft member to enhance the structural integrity of the stent graft. The increased bonding surface area also facilitates minimizing the thickness of the tape member. In addition, the tape member is arranged in a helical configuration according to the embodiment illustrated in **Figure 14** (helically arranged tape member **1406**). As shown, the tape member may be constructed with a constant width and arranged with uniform spacing between turns. Tape member **1406** not only covers the apices in the first direction of each of the undulations, but also covers a portion of each undulation. In another embodiment, there can be several tape members on a stent graft, which serves the same function as described above. A non-limiting reason to have several tape members on a stent graft is if there is a disruption in the stent pattern, such as changing the stent pattern to make room for a portal for a side-branch device, as depicted in **Figure 12**, **1206**, **Figure 14**, **1408**, and **Figure 1B**, **121**. In another embodiment, said tape member does not overlap an adjacent row of undulating stent members when the stent graft is not compressed. Although the Examples and Figures show an embodiment wherein apices in the first direction of the each of the undulations are attached to the stent graft by the tape member, said apices in the second direction may also be attached to the stent graft while the apices in the first direction are not attached.

It has been found that the width of the tape member can affect the flexibility of the stent graft. The wider the tape member, the less flexible the stent graft will become. Thus, in one embodiment said tape member covers about 10%, about 20% about 30%, about 40%, about 50%, about 60%, about 70%, about 80% of undulation height **1312** (**Figure 13**). In another embodiment, the full width of said tape member is adhered to said stent and graft. In another embodiment, said tape member does not extend to or touch an adjacent row of the undulating stent members, *e.g.* when not compressed or partially compressed. In another embodiment, the width of said unidirectional pleats is the same as the width of the tape member. Although the tape member can cover a portion of each undulation, including confining the apices in the first direction to the graft, as discussed above, apices in the second direction of the undulation are not confined relative to the graft (*e.g.* **816** in **Figure 8**). This construction allows for the formation of pleats where the stent graft is compressed. Pleats can be fully circumferential when the stent graft is compress longitudinally, as depicted in **Figure 9**, or in the inner meridian of a bend, as depicted in **Figure 11**. In another embodiment, said unidirectional circumferential pleats are formed when initially compressed. In other words, no further manipulation of the stent graft is required to create said unidirectional circumferential pleats. In another embodiment, said unidirectional circumferential pleat are formed *in-vivo* when deployed. In another embodiment said pleats will be formed in the inner meridian *in-vivo* when said stent graft is deployed. Said stent graft of the invention can conform, as describe above, to the aortic arch or other tortuous, curved or bent body lumen. In another embodiment, tape member (or separate pieces thereof) also surrounds the terminal end portions of the stent graft to secure the terminal portions of the graft member to the support structure formed by stent member.

In another embodiment of the invention, the tape member is made from expanded PTFE (ePTFE) coated with an adhesive. In another embodiment, said adhesive is a thermoplastic adhesive. In another embodiment, said thermoplastic adhesive is fluorinated ethylene propylene (FEP). In this embodiment, the FEP-coated side faces toward and contacts the exterior surface of the stent and graft, thus attaching the stent to the graft. Although a particular tape member configuration and pattern has been illustrated and described, other configuration and/or patterns may be used without departing from the scope of the present invention.

In another embodiment of the invention, said stent graft of the invention comprises one or more radiopaque metallic fibers, such as gold, platinum, platinum-tungsten, palladium, platinum-iridium, rhodium, tantalum, or alloys or composites of these metals that may be incorporated into the device, particularly, into the graft, to allow fluoroscopic visualization of the device.

In another embodiment of the invention, said stent graft of the invention comprises optional sealing cuffs **808** and **812** as shown in **Figure 8**. Said sealing cuff comprises a cuff which has a first cuff end secured to outer surface of the stent graft **800** and a second cuff end at least a portion of which is unsecured to form a flange. In this configuration, the flange forms a one-way valve that circumferentially surrounds the stent graft **800** and occludes flow around the stent graft. In one embodiment, said sealing cuff is positioned around the first end portion **806** of the stent graft **800**. In another embodiment, said sealing cuff is positioned around the second end portion **810** of the stent graft **800**. In another embodiment, said sealing cuff is positioned around the first end portion **806** and the second end portion **810** of the stent graft **800**. In another embodiment, sealing cuffs (**808**, **812**) comprise a hydrophilic material, preferably a hydrophilic polymer or gel-foam, which expands when exposed to water, such as in blood or other water-containing body fluids. In another embodiment, said sealing cuffs **808** and **812** can comprise the materials described above. A description of sealing cuffs is found in U.S. Patent 6,015,431, incorporated by reference herein in its entirety for all purposes.

This invention is further illustrated by the following Examples which should not be construed as limiting. The contents of all Figures and references are incorporated herein by reference.

While particular embodiments of the present invention have been illustrated and described herein, the present invention should not be limited to such illustrations and descriptions. It should be apparent that changes and modifications may be incorporated and embodied as part of the present invention within the scope of the following claims.

### EXAMPLE 1

### Construction of a Highly conformable Stent graft

A flexible stent graft was assembled having the general configuration as shown in **Figure 8**.

The stent graft was fabricated by initially extruding and expanding a tube of polytetrafluoroethylene (PTFE) to form a base tube. The base tube had a length of about 60 mm, a wall thickness of about 0.06 mm and a diameter of about 26 mm. The base tube had a substantial fibril orientation in the longitudinal direction so that the tube was relatively strong in the longitudinal direction while being relatively weak in the radial direction. The base tube was radially stretched over a mandrel having a diameter of about 31 mm.

To provide resistance to fluid permeation and to enhance the radial strength of the base tube, a film of densified ePTFE was wrapped over the base tube. The film was a thin, strong fluoropolymer; a particularly preferred material for this application is a non-porous ePTFE provided with an adhesive coating of thermoplastic fluorinated ethylene propylene (FEP), referred to hereinafter as "substantially impermeable ePTFE/FEP insulating tape". The FEP was oriented down against the base tube. EPTFE is well known in the medical device arts; it is generally made as described by U.S. Patents 3,953,566 and 4,187,390 to Gore. The particular tape described herein is slit from a substantially non-porous ePTFE/FEP film having a thickness of about 0.0064 mm, an isopropyl bubble point of greater than about 0.6 MPa, a Gurley No. (permeability) of greater than 60 (minute/1 square inch/100cc ); (or 60 (minute/6.45 square cm/100cc)), a density of 2.15g/cc and a tensile strength of about 309 MPa in the length direction (i.e., the strongest direction). The film had a width of about 19 mm (0.75") with four passes helically wrapped with a pitch angle of about 86°.

To further enhance the radial strength of the base tube and to provide an open structure bonding layer, an additional layer of film was applied. The ePTFE film had high degree strength in the longitudinal direction and had a very open microstructure. The open microstructure enhanced the subsequent FEP/ePTFE bonding of a stent frame to the graft. The film had a thickness of about 2.5 microns (0.0001") and a width of about 25.4 mm (1.0"). Eight helically wrapped layers were applied with a pitch angle of about 83°.

The mandrel and wrapped films were then heat treated in an air convection oven to bond the films together.

A stent frame was then formed by winding a Nitinol wire onto a mandrel having protruding pins. A "flat or unrolled" drawing of the cylindrical mandrel is shown in **Figure 12**. Shown is an overall winding pattern **1200**, detailing a first end portion **1202** and a second end portion **1204**. Also shown is an optional "side branch portal" configuration **1206** that can be incorporated into the overall pattern if a branch portal is desired. The generic single circumference winding pattern shown as 1206 can replace the optional side branch pattern **1206** if desired.

Shown in **Figure 13** is a single circumference winding pattern shown as **1206**. The pattern includes a linear pitch **1300**, a pin diameter **1302**, a wire diameter **1304**, a wire apex angle **1306**, a circumference **1308** and an apex to base half frequency **1310**. The pattern shown was repeated along the stent length with the exception of the first and second end portions previously shown in **Figure 12** (**1202**, **1204**). The optional side branch portal configuration (**Figure 12**, **1206**) was not incorporated.

The stent frame was formed according to the following dimensions as defined in **Figure 13**: the linear pitch **1300** was about 9.7 mm (0.383"), the pin diameter **1302** was about 1.6 mm (0.063"), the wire diameter **1304** was about 0.5 mm (0.0195"), the wire apex angle **1306** was about 50.4 degrees, the circumference **1308** was about 97.3 mm (3.83") and the apex to base half frequency **1310** was about 5.3 mm (0.21").

The mandrel with the wound wire was then heat treated in an air convection oven as is commonly known in the art (*e.g*. see U.S. Patent 6,352,561 to Leopold), and then quenched in room temperature water.

The wire stent was the removed from the winding mandrel. The wire ends (shown in **Figure 12**, **1202** and **1204**) were trimmed and tied together with high temperature fibers as shown in **Figure 14**, **1402** and **1404**. The amplitude of the nested pair is longer than the adjacent apexes so that when the wires are nested the nested wires to not create an adversely high strained region (see Figure 14, 1410 and 1412). The stent was partially joined to the wrapped tube by melting the underlying FEP adjacent to portions of the stent wire using a soldering iron. A final layer of an ePTFE tape, laminated with FEP, was wrapped over the wire stent according to the pattern depicted in **Figure 14** and placed in an oven to bond the film to the underlying graft, thus securing the stent to the graft.

Shown in **Figure 14** is a stent graft **1400** having an undulating, helical wire stent **802** surrounding a graft material **804**. The stent is attached to the graft material by a helically applied tape member **1406**. As shown, the first edge of the helically applied tape member **1406** covers the opposing first apices **814** of the wire stent. An optional wrapping pattern section **1408** can be incorporated if a side branch portal is desired. The tape **1406** was an ePTFE/FEP laminate having a width of about 5.5 mm (0.215") and a thickness of about 10 microns (0.0004"). The tape was partially joined to the wrapped tube by melting the underlying FEP adjacent to portions of the stent wire using a soldering iron. A sacrificial compression tape was helically wrapped onto the stent graft. The compression tape was about 51 mm (2") wide, about 0.5 mm (0.02") thick and was wrapped with an approximate 50% overlap. An additional sacrificial film was wrapped to assist in the subsequent heat treatment compression step. This film was an ePTFE tape having a longitudinal fibril/strength orientation, a thickness of about 2.5 microns (0.0001") and a width of about 51 mm (2"). Five passes were applied with an approximate 50% overlap between the film layers.

The assembly was then heat treated in an air convection oven to bond the film layers together (as essentially described in U.S. Patent 6,352,561 to Leopold). During this heat treat cycle, the film compressed down against the mandrel causing the melted FEP to flow into the underlying film layers, joining the graft layers together along with the wire stent. After cooling the sacrificial film compression layers were removed, the ends of the graft material were trimmed to length and the stent graft was removed from the mandrel. The resulting stent graft is depicted in **Figure 8**, with the exception of the optional sealing cuffs (**Figure 8**, **806**, **810**).

### EXAMPLE 2

### Construction of a Highly Conformable Stent graft having an Integral Side Branch Portal

Referring to Figures 2A and 2B, a metallic mandrel **200** was fabricated having a slot **202** formed into one end of the mandrel. The slot **202** terminates onto a back wall **204**. The mandrel had a diameter of about 31 mm and the slot was about 12.5 mm wide, by about 10 mm deep and about 13 cm long. As shown in **Figure 2B**, an inner tube **206** was radially stretched onto the mandrel **200**, covering a portion of the mandrel groove **202**. The inner tube was an extruded and expanded tube of polytetrafluoroethylene (PTFE). The inner tube had a length of about 60 mm, a wall thickness of about 0.06 mm and a diameter of about 26 mm. The inner tube had a substantial fibril orientation in the longitudinal direction so that the tube was relatively strong in the longitudinal direction while being relatively weak in the radial direction.

As shown in **Figure 3A** the mandrel **200** was covered by the inner tube **206**. The inner tube was cut, forming a slit **300** at the back wall **204** of the mandrel groove **202**.

To further strengthen the inner tube (**Figure 3A**, **206**), two additional polymeric sheets were added onto the inner tube prior to the insertion of the secondary stent assembly. The strengthening layers were then deformed into the mandrel groove, providing additional support to the inner tube/secondary stent assembly. The strengthening layers comprised densified ePTFE provided with an adhesive coating of thermoplastic fluorinated ethylene propylene (FEP) referred to hereinafter as "substantially impermeable ePTFE/FEP insulating tape". The FEP of the strengthening layers was oriented towards the base tube. EPTFE is well known in the medical device arts; it is generally made as described by U.S. Patents 3,953,566 and 4,187,390 to Gore. The particular strengthening layers described herein were slit from a substantially non-porous ePTFE/FEP film having a thickness of about 0.0064 mm, an isopropyl bubble point of greater than about 0.6 MPa, a Gurley No. (permeability) of greater than 60 (minute/1 square inch/100 cc ); (or 60 (minute/6.45 square cm/100cc)), a density of 2.15g/cc and a tensile strength of about 309 MPa in the length direction (i.e., the strongest direction). The first strengthening layer was about 25 mm wide by about 25 mm long and was centered over the mandrel slot about 15 mm from the slot back wall (towards the end of the mandrel). The second strengthening layer was about 25 mm wide and about 40 mm long and was centered over the mandrel slot abutting the slot back wall **204**.

As shown in **Figure 3B**, a secondary stent assembly **302** was aligned to the mandrel groove **202**, mandrel back wall **204**, strengthening layers and inner tube slit **300**. A first support segment (subsequently described) was placed into the secondary stent assembly and the secondary stent assembly **302** (with the first support segment) was then inserted into the mandrel groove **202**, deforming the inner tube **206** (and strengthening layers) into the mandrel groove. The back wall **204** defined the opening in the innermost tube (**130**, **Figure 1E**).

To control the deformed shape of the inner tube, a support segment was placed into the secondary stent assembly and into the mandrel groove, as depicted in **Figures 4A** and **4B**. Shown in **Figure 4A** is a side view schematic of the mandrel **200**, the groove **202** and the groove back wall **204**. Shown in **Figure 4B** is a side view schematic of the mandrel **200**, mandrel groove **202** and inner tube **206**. The secondary stent assembly **302** was placed over a first support segment **400** having an end formed to mate to the mandrel groove back wall **204**. The opposing end of the first support segment had a tapered or angulated wall as depicted in **Figure 4B**. A second support segment **402** was placed into the mandrel groove **202** under the inner tube **206**. The second support structure had flat walls and was used to hold of the first support structure **400** in place. The inner tube **206** is shown deformed into the mandrel groove **202**. The inner tube **206** is also shown having a tapered, beveled or angulated wall portion **404** formed by the angulated wall of support segment **400**.

A secondary stent assembly was assembled as outlined in **Figures 5A** through **5D**. As shown in **Figures 5A** and **5B**, a polymeric tube **502** was slip-fit onto a mandrel **500**. The tube was formed from a film of the same material used for the strengthening layers as previously described. The film was helically wrapped onto a mandrel having a diameter of about 8 mm with the FEP layer oriented away from the mandrel. The wrapped mandrel was then heat set to fuse the FEP/ePTFE layers forming a tube. An undulating wire was formed into a ring stent **506** by winding the wire onto a mandrel with protruding pins. The diameters of the mandrel and pins along with the locations of the pins dictated the final configuration of the ring stent. The wire was Nitinol and had a diameter of about 0.15 mm. The undulating stent pattern had an apex to apex length of about 5 mm. After the wire was wound onto the mandrel, the mandrel and wire were heat treated and quenched in room temperature water to set the shape of the stent. The wire was then removed from the mandrel. The ends of the wire were joined together with a section of polymeric heat shrink tubing, forming ring stent **506**. Two of these ring stents **506** were then placed onto the polymeric tube **502**. Radiopaque gold marker bands **504** were then placed onto the polymeric tube **502**.

Next, as shown in **Figure 5C**, one end of the polymeric tube **502** was inverted and drawn over the wire ring stents **506** and radiopaque marker bands **504**. The mandrel, polymeric tube, ring stents and radiopaque bands were then heat treated to bond the components together into a stent assembly. The assembly was removed from the mandrel and trimmed to length, forming a secondary stent assembly **302** as shown in **Figure 5D**. The secondary stent assembly was then placed onto a first support segment **400**.

As previously described (**Figure 4B**), the secondary stent (or secondary stent assembly) and the first support assembly were then inserted into the mandrel groove **202**, deforming the inner tube **206** into the mandrel groove. A second support segment **402** was placed into the mandrel groove **202** under the inner tube **206**. The assembly shown in **Figure 4B** was then covered with an outer support film. The support was formed from a film of the same material used for the strengthening layers as previously described. The film was about 30 mm wide by about 27 mm wide and was centered over the mandrel slot about 6 mm behind the slot back wall (away from the mandrel end). The FEP layer was oriented down toward the mandrel.

To provide resistance to fluid permeation and to enhance the radial strength of the base tube, a film of densified ePTFE was wrapped over the base tube. The film was a thin, strong fluoropolymer; a particularly preferred material for this application is a non-porous ePTFE provided with an adhesive coating of thermoplastic fluorinated ethylene propylene (FEP), referred to hereinafter as "substantially impermeable ePTFE/FEP insulating tape". The FEP was oriented down against the base tube. EPTFE is well known in the medical device arts; it is generally made as described by U.S. Patents 3,953,566 and 4,187,390 to Gore. The particular tape described herein is slit from a substantially non-porous ePTFE/FEP film having a thickness of about 0.0064 mm, an isopropyl bubble point of greater than about 0.6 MPa, a Gurley No. (permeability) of greater than 60 (minute/1 square inch/100 cc ); (or 60 (minute/6.45 square cm/100 cc)), a density of 2.15g/cc and a tensile strength of about 309 MPa in the length direction (i.e., the strongest direction). The film had a width of about 19 mm (0.75") with four passes helically wrapped with a pitch angle of about 86°.

To further enhance the radial strength of the base tube and to provide an open structure bonding layer, an additional layer of film was applied. The ePTFE film had a high degree strength in the longitudinal direction and had a very open microstructure. The open microstructure enhanced the subsequent FEP/ePTFE bonding of a stent frame to the graft. The film had a thickness of about 2.5 microns (0.0001") and a width of about 25.4 mm (1.0"). Eight helically wrapped layers were applied with a pitch angle of about 83°.

The mandrel and wrapped films were then heat treated in an air convection oven to bond the film layers together.

A stent frame was then formed by winding a Nitinol wire onto a mandrel having protruding pins. A "flat or unrolled" drawing of the cylindrical mandrel is shown in **Figure 12**. Shown is an overall winding pattern **1200**, detailing a first end portion **1202** and a second end portion **1204**. Also shown is a "side branch portal" configuration **1206** that was incorporated into the overall pattern to form a branch portal.

Shown in **Figure 13** is a single circumference winding pattern shown as **1206**. The pattern includes a linear pitch **1300**, a pin diameter **1302**, a wire diameter **1304**, a wire apex angle **1306**, a circumference **1308** and an apex to base half frequency **1310**. The pattern shown was repeated along the stent length with the exception of the first and second end portions previously shown in **Figure 12** (**1202**, **1204**). The optional side branch portal configuration (**Figure 12**, **1206**) was incorporated.

The stent frame was formed according to the following dimensions as defined in **Figure 13**: the linear pitch **1300** was about 9.7 mm (0.383"), the pin diameter **1302** was about 1.6 mm (0.063"), the wire diameter **1304** was about 0.5 mm (0.0195"), the wire apex angle **1306** was about 50.4 degrees, the circumference **1308** was about 97.3 mm (3.83") and the apex to base half frequency **1310** was about 5.3 mm (0.21").

The mandrel with the wound wire was then heat treated in an air convection oven as is commonly known in the art and then quenched in room temperature water.

The wire stent was the removed from the winding mandrel. The wire ends (shown in **Figure 12**, **1202** and **1204**) were trimmed and tied together with high temperature fibers as shown in **Figure 14**, **1402** and **1404**. The wire stent was then placed onto the previously film wrapped tube/mandrel. The stent was partially joined to the wrapped tube by melting the underlying FEP adjacent to portions of the stent wire using a soldering iron. A final layer of an ePTFE tape, laminated with FEP was wrapped over the wire stent according to the pattern depicted in **Figure 14**.

Shown in **Figure 14** is a stent graft **1400** having an undulating, helical wire stent **802** surrounding a graft material **804**. The stent was attached to the graft material by a helically applied tape member **1406**. As shown, the first edge of the helically applied tape member **1406** covers the opposing first apices **814** of the wire stent. The wrapping pattern section **1408** was incorporated to form a side branch portal. The tape **1406** was an ePTFE/FEP laminate having a width of about 5.5 mm (0.215") and a thickness of about 10 microns (0.0004"). The tape was partially joined to the wrapped tube by melting the underlying FEP adjacent to portions of the stent wire using a soldering iron. A sacrificial compression tape was helically wrapped onto the stent graft. The compression tape was about 51 mm (2") wide, about 0.5 mm (0.02") thick and was wrapped with an approximate 50% overlap. An additional sacrificial film was wrapped to assist in the subsequent heat treatment compression step. This film was an ePTFE tape having a longitudinal fibril/strength orientation, a thickness of about 2.5 microns (0.0001") and a width of about 51 mm (2"). Five passes were applied with an approximate 50% overlap between the film layers.

The assembly was then heat treated in an air convection oven to bond the film layers together. During this heat treat cycle, the film compressed down against the mandrel causing the melted FEP to flow into the underlying film layers, joining the graft layers together along with the wire stent. After cooling the sacrificial film compression layers were removed, the ends of the graft material were trimmed to length and the stent graft was removed from the mandrel. The resulting stent graft is depicted in **Figure 8**, with the exception of the optional sealing cuffs (**Figure 8**, **806**, **810**).

The invention further relates to the following numbered aspects.
In a first aspect there is provided a multi-lumen stent graft comprising:
   a primary lumen defined by a graft composed of an innermost tube with an opening and an outermost tube with an opening, said graft being supported by a primary stent; and
   a secondary lumen disposed between the innermost tube and outermost tube of said graft;
   wherein said secondary lumen is in fluid communication through said openings.
In a second aspect there is provided the stent graft of the first aspect, wherein said secondary lumen comprises a secondary stent or stent assembly.
In a third aspect there is provided the stent graft of the first aspect, wherein said outermost tube comprises ePTFE.
In a fourth aspect there is provided the stent graft of the first aspect, wherein said innermost tube comprises ePTFE.
In a fifth aspect there is provided the stent graft of the first aspect, wherein said primary stent is attached to said graft by coupling member.
In a sixth aspect there is provided the stent graft of the second aspect, wherein said primary stent comprises a double W which creates a region for a side branch portal.
In a seventh aspect there is provided the stent graft of the second aspect, wherein said primary stent comprises undulations.
In an eighth aspect there is provided the stent graft of the seventh aspect, wherein said undulations are in-phase.
In a ninth aspect there is provided the stent graft of the third aspect, wherein said secondary stent is covered.
In a tenth aspect there is provided the stent graft of the ninth aspect, wherein said secondary stent covering comprises ePTFE.
In an eleventh aspect there is provided the stent graft of the second aspect, wherein a portion of the said secondary stent or stent assembly abuts against a portion of the innermost tube.
In a twelfth aspect there is provided the stent graft of the first aspect, wherein said stent graft comprises cuffs on both ends of the stent graft.
In a thirteenth aspect there is provided the stent graft of the first aspect, wherein said secondary lumen can accept another smaller stent graft.
In a fourteenth aspect there is provided the stent graft of the thirteenth aspect, wherein said other smaller stent graft locks into said secondary lumen.
In a fifteenth aspect there is provided a stent graft for implantation in a bifurcated body lumen having a main branch vessel and a side branch vessel, wherein the stent graft comprises:
   a graft, said graft composed of an innermost tube with an opening and an outermost tube with an opening, said graft extending along a longitudinal axis from a distal end to a proximal end and defining a main lumen extending therethrough, said graft being supported by a primary stent; and
   a secondary lumen disposed between the innermost tube and outermost tube of said graft, said secondary lumen portion positioned between the distal and proximal ends of said graft, wherein said secondary lumen is in fluid communication through said openings of said innermost and outermost tubes.
In a sixteenth aspect there is provided the stent graft of the fifteenth aspect, wherein said primary stent is a self expanding stent.
In a seventeenth aspect there is provided the stent graft of the sixteenth aspect, wherein said self expanding stent comprises a titanium-nickel alloy.
In an eighteenth aspect there is provided the stent graft of the fifteenth aspect, wherein said primary stent comprises a single continuous wire helically wrapped around said graft.
In a nineteenth aspect there is provided the stent graft of the eighteenth aspect, wherein said single continuous wire comprises undulations.
In a twentieth aspect there is provided the stent graft of the eighteenth aspect, wherein said single continuous wire comprises a double W that creates a region for a side branch portal.
In a twenty-first aspect there is provided the stent graft of the nineteenth aspect wherein said undulating wire comprises multiple turns of said undulations, and each turn of said undulating wire comprises multiple apexes, with undulation in one turn generally in-phase with undulation in an adjacent turn.
In a twenty-second aspect there is provided the stent graft of the twenty-first aspect, wherein said undulations are U shaped.
In a twenty-third aspect there is provided the stent graft of the twenty-first aspect, wherein said undulations are V shaped.
In a twenty-fourth aspect there is provided the stent graft of the twenty-first aspect, wherein said undulations are ovaloid shaped.
In a twenty-fifth aspect there is provided the stent graft of the twenty-first aspect, wherein said undulations are sinusoidal shaped.
In a twenty-sixth aspect there is proved the stent graft of the fifteenth aspect, wherein said primary stent is attached to said graft.
In a twenty-seventh aspect there is provided the stent graft of the sixteenth aspect, wherein said primary stent is attached to said graft by a ribbon or tape.
In a twenty-eighth aspect there is provided the stent graft of the twenty-seventh aspect, wherein said ribbon or tape is adhered to a portion of said stent and a portion of said graft.
In a twenty-ninth aspect there is provided the stent graft of the twenty-seventh aspect, wherein said ribbon or tape is arranged in a helical configuration with multiple turns.
In a thirtieth aspect there is provided the stent graft of the twenty-ninth aspect, wherein said ribbon or tape is arranged in a helical configuration with multiple turns, each turn being spaced from an adjacent turn.
In a thirty-first aspect there is provided the stent graft of the thirtieth aspect, wherein said spacing between said turns is uniform.
In a thirty-second aspect there is provided the stent graft of the twenty-seventh aspect, wherein said ribbon covers a portion of said undulation.
In a thirty-third aspect there is provided the stent graft of the twenty-seventh aspect, wherein said primary stent comprises undulations each which comprise an apex portion and a base portion and said ribbon or tape is attached to said primary stent such that the ribbon or tape is placed along the base portion of each of the undulations thus confining the base portion of the undulations to the graft and wherein the apex portion of the undulation is not confined.
In a thirty-fourth aspect there is provided the stent graft of the twenty-seventh aspect, wherein said stent graft can nest.
In a thirty-fifth aspect there is provided a method of making a multi-lumen stent graft comprising:
   covering a first mandrel that comprises a groove and a back wall of said groove with an innermost polymeric tube;
   slitting said innermost polymeric tube along said back wall of said groove;
   placing a second mandrel into said groove of the first mandrel and aligning said second mandrel with the back wall of the groove, deforming said innermost polymeric tube;
   placing an outermost polymeric tube over said innermost polymeric tube; and
   cutting an opening in said outermost polymeric tube over said second smaller mandrel;
   wherein said outermost polymeric tube and innermost polymeric tube comprise a graft member.
In a thirty-sixth aspect there is provided the method of the thirty-fifth aspect, wherein a primary stent is placed over said graft member.
In a thirty-seventh aspect there is provided the method of the thirty-sixth aspect, wherein said primary stent is attached to said graft member by coupling member.
In a thirty-eighth aspect there is provided the method of the thirty-sixth aspect, wherein said primary stent comprises undulations.
In a thirty-ninth aspect there is provided the method of the thirty-eighth aspect, wherein said undulations are in-phase.
In a fortieth aspect there is provide the method of the thirty-fifth aspect, wherein said outermost polymeric tube comprises ePTFE.
In a forty-first aspect there is provided the method of the thirty-fifth aspect, wherein said innermost polymeric tube comprises ePTFE.
In a forty-second aspect there is provided the method of the thirty-fifth aspect, wherein there is a secondary stent or stent assembly around said second mandrel.
In a forty-third aspect there is provided the method of the forty-second aspect, wherein said secondary stent or stent assembly abuts at least a portion of the innermost tube.
In a forty-fourth aspect there is provided the method of the thirty-fifth aspect, wherein the slitting of the innermost polymeric tube and making of an opening in the outermost polymeric tube creates a secondary lumen that is in fluid communication through said openings.
In a forty-fifth aspect there is provided a stent graft comprising:
   a graft being supported by a stent, wherein said stent comprises undulations each which comprise apices in opposing first and second directions; and
   a tape member, comprising first and second longitudinal edges, attached to said stent and to said graft such that the first tape edge substantially covers the apices in the first or second direction of the each of the undulations, thus confining the apices in the first or second direction of the undulations to the graft and wherein the apices in the first or second direction of the undulation are not confined relative to the graft;
   wherein said graft forms circumferentially oriented unidirectional pleats when longitudinally compressed and said apices in the first or second direction of said undulation are positioned under an adjacent pleat when compressed.
In a forty-sixth aspect there is provided the stent graft of the forty-fifth aspect, wherein said apices in the first direction are confined to the graft and the apices in the second direction are not confined relative to the graft.
In a forty-seventh aspect there is provided the stent graft of the forty-fifth aspect, wherein said apices in the second direction are confined to the graft and the apices in the first direction are not confined relative to the graft.
In a forty-eighth aspect there is provided the stent graft of the forty-fifth aspect, wherein said stent is formed from a single continuous wire helically wrapped around said graft.
In a forty-ninth aspect there is provided the stent graft of the forty-fifth aspect, wherein said stent is a self-expanding stent.
In a fiftieth aspect there is provided the stent graft of the forty-fifth aspect, wherein said stent is made from Nitinol.
In a fifty-first aspect there is provided the stent graft of the forty-fifth aspect, wherein said stent is a balloon expandable stent.
In a fifty-second aspect there is provided the stent graft of the forty-fifth aspect, wherein said undulations have a sinusoidal shape.
In a fifty-third aspect there is provided the stent graft of the forty-fifth aspect, wherein said unidirectional pleats are formed *in- vivo* after deployment.
In a fifty-fourth aspect there is provided the stent graft of the forty-fifth aspect, wherein said graft comprises polytetrafluoroethylene.
In a fifty-fifth aspect there is proved the stent graft of the fifty-fourth aspect, wherein said polytetrafluoroethylene is expanded.
In a fifty-sixth aspect there is provided the stent graft of the forty-fifth aspect, wherein said tape member comprises polytetrafluoroethylene.
In a fifty-seventh aspect there is provided the stent graft of the forty-fifth aspect, wherein said tape member further comprises a thermoplastic adhesive.
In a fifty-eighth aspect there is provided the stent graft of the fifty-seventh aspect, wherein said thermoplastic adhesive is FEP.
In a fifty-ninth aspect there is provided the stent graft of the forty-fifth aspect, wherein said stent graft comprises at least one sealing cuff.
In a sixtieth aspect there is provided the stent graft of the forty-fifth aspect, wherein said stent graft comprises at least one radiopaque marker.
In a sixty-first aspect there is provided the stent graft of the forty-fifth aspect, wherein said stent graft can bend to at least 90° without kinking *in-vivo* when deployed.
In a sixty-second aspect there is provided the stent graft of the sixty-first aspect, wherein said stent graft is placed into a body lumen with blood flow direction going with the pleats to minimize flow disruption and turbulence.

It will be apparent to those skilled in the art that various modifications and variation can be made in the present invention without departing from the spirit or scope of the invention. Thus, it is intended that the present invention cover the modifications and variations of this invention provided they come within the scope of the appended claims and their equivalents.

## Claims

1. A stent graft comprising:
a graft having an inward-facing surface and an outward-facing surface;
a stent having undulations defining a first set of apices extending in a first direction and a second set of apices extending in an opposing second direction; and
a tape member extending helically about and being fixedly secured to the graft such that a portion of the tape member covers the first set of apices of the stent to form a series of confined apices in the first direction and a series of unconfined apices in the second direction, whereby the stent graft is configured to be longitudinally compressed to form circumferentially oriented unidirectional pleats along one of the inward-facing and outward-facing surfaces of the graft with the confined apices positioned under an adjacent pleat.

2. The stent graft of claim 1, wherein the tape member is bonded at least in part to the stent by an adhesive.

3. The stent graft of any one of the preceding claims, wherein a portion of the tape member in each row substantially covers the apices of the stent in an adjacent row thereby forming the series of confined apices in the first direction and the series of unconfined apices in the second direction.

4. The stent graft of any one of the preceding claims, wherein the series of confined apices are in the first direction relative to the graft and the series of unconfined apices are in the second direction relative to the graft.

5. The stent graft of any one of the preceding claims, wherein the series of confined apices are in the second direction relative to the graft and the series of unconfined apices are in the first direction relative to the graft.

6. The stent graft of any one of the preceding claims, wherein the stent is formed from a single continuous wire helically wrapped around the graft.

7. The stent graft of any one of the preceding claims, wherein the stent is a self-expanding stent or a balloon expandable stent.

8. The stent graft of any one of the preceding claims, wherein the undulations have a sinusoidal shape.

9. The stent graft of any one of the preceding claims, wherein the pleats are adapted to form in-vivo after deployment.

10. A method of forming a stent graft comprising:
providing a graft having an inward-facing surface and an outward-facing surface;
positioning a stent with the graft, the stent having undulations defining apices that extend in opposing first and second directions; and
fixedly securing a member to the outward-facing surface of the graft and a portion of the stent, wherein the stent graft is configured to form pleats including folded portions of the graft when compressed, wherein the apices in the first direction of the undulation are positioned under an adjacent pleat when the stent graft is compressed.

11. The method of claim 10, wherein the graft is configured to form pleats when longitudinally compressed.

12. The method of claim 10 or 11, wherein the graft is configured to form pleats on one of an inner median and an outer meridian of the graft in response to the graft being bent.

13. The method of claim 12, wherein the graft is configured to form pleats on the inner median in response to compression of the inner median and the graft is configured to remain un-pleated along the outer median, and the graft forms a bent shape in response to the compression.

14. The method of any one of claims 10 to 13, wherein the stent graft comprises at least one sealing cuff.

15. The method of any one of claims 10 to 14, wherein the stent graft can bend to 90° without kinking in-vivo when deployed.
